# FASCICULE DE BREVET EUROPEEN

(11) **EP 1 202 765 B1**
(45) Date de publication et mention de la délivrance du brevet: **24.11.2004**
(21) Numéro de dépôt: 00949592.0
(22) Date de dépôt: 30.06.2000
(51) Int. Cl.: A61M 5/30

(54) **SERINGUE SANS AIGUILLE MUNIE D'UN SYSTEME DE DECLENCHEMENT PIEZO-ELECTRIQUE**
NADELLOSE SPRITZE MIT PIEZOELEKTRISCHER AUSLÖSEEINRICHTUNG
NEEDLELESS SYRINGE PROVIDED WITH A PIEZOELECTRIC TRIGGERING SYSTEM

(30) Priorité: 16.07.1999 FR 9909254
(43) Date de publication de la demande: 08.05.2002
(73) Titulaire: CROSSJECT, 75181 Paris Cedex 04 (FR)
(72) Inventeur: ALEXANDRE, Patrick, F-70100 Gray (FR); BROUQUIERES, Bernard, F-83100 Toulon (FR); MIKLER, Claude, F-21000 Dijon (FR); SIMONET, Louis, F-83130 La Garde (FR)
(74) Mandataire: Waligorski, Carol
(86) Numéro de dépôt international: PCT/FR2000/001849
(87) Numéro de publication internationale: WO 2001/005452

(56) Documents cités:
- EP-A- 0 853 952
- US-A- 3 802 430
- US-A- 4 089 334

## Description

Le domaine technique de l'invention est celui des seringues sans aiguille destinées à injecter, à travers la peau, des particules solides ou liquides de principe actif à usage thérapeutique.

En effet, l'invention se rapporte, d'une part, à une seringue sans aiguille fonctionnant à partir d'un dispositif d'initiation faisant intervenir un dispositif de déclenchement associé à une charge pyrotechnique et, d'autre part, à ce dispositif de déclenchement adapté à la mise à feu d'une charge pyrotechnique logée dans un objet léger, de petite taille et devant être actionné manuellement, cet objet étant notamment une seringue sans aiguille.

La solution proposée par l'invention prône l'utilisation d'un cristal piézo-électrique comme pièce centrale du dispositif de déclenchement de la seringue sans aiguille.

Il s'avère que dans le domaine des seringues sans aiguille, aucun brevet ne se rapporte à la mise en oeuvre d'un cristal piézo-électrique dans le dispositif de déclenchement de telles seringues. On peut toutefois citer la demande de brevet européen EP 0 853 952 qui décrit un boitier d'allumage autonome pouvant s'emboîter sur des seringues sans aiguille et pouvant être utilisé plusieurs fois. Il est seulement fait allusion que ce boitier d'allumage autonome pouvait faire intervenir un cristal piézo-électrique. Mais en aucune façon ce dispositif d'allumage ne fait partie intégrante de la seringue sans aiguille comme l'impose l'invention.

En revanche, l'utilisation d'un cristal piézo-électrique pour initier une charge pyrotechnique dans des objets tels que, par exemple, des fusées électriques, des cartouches de sautages ou des amorces électriques de fusées est connue et a fait l'objet de plusieurs brevets. On peut citer, entre autres, le brevet français FR 2 665 253 qui décrit un dispositif de mise à feu piézo-électrique à fil résistant d'un ensemble pyrotechnique. Ce dispositif est apte à être actionné manuellement par l'intermédiaire d'un bouton poussoir qui permet de déclencher la mise en mouvement d'une masselotte destinée à impacter le cristal piézo-électrique. EP-A1-0 853 952 montre les caracteristiques du preambule de la revendication 1.

Les seringues sans aiguille recherchées par l'homme du métier doivent être dotées d'un dispositif de déclenchement pouvant être actionné manuellement et permettant de s'affranchir d'une source d'activation trop énergétique ou trop encombrante tout en restant fiable et performant.
La seringue sans aiguille selon l'invention répond à ces exigences.

L'objet de la présente invention concerne une seringue sans aiguille munie d'un dispositif d'initiation comprenant un dispositif de déclenchement et une charge pyrotechnique, caractérisée en ce que le dispositif de déclenchement comporte un cristal piézo-électrique selon la revendication 1 ainsi qu'un dispositif de déclenchement pour seringue selon la revendication 12.

Le dispositif de déclenchement comprend un organe de déclenchement relié à un moyen de percussion du cristal piézo-électrique.
De façon préférentielle, le cristal piézo-électrique est positionné dans la seringue entre le moyen de percussion et la charge pyrotechnique à allumer, et il est inséré de sorte que, pendant le fonctionnement de la seringue, il assure l'étanchéité de la partie amont de la seringue constituée par l'organe de déclenchement et le moyen de percussion, vis à vis de sa partie aval comprenant la charge pyrotechnique, le principe actif à injecter et une buse d'injection.

Selon l'invention, le moyen de percussion comprend une languette maintenue sous contrainte élastique par blocage contre une butée et l'organe de déclenchement est apte à déplacer ladite languette pour la libérer, de manière à venir impacter le cristal piézo-électrique.
Préférentiellement, la languette est réalisée dans un matériau présentant une élasticité élevée, comme par exemple l'acier à ressort ou le bronze à ressort.
De façon avantageuse, la languette se termine par une masselotte pour accroître la force d'impact sur le cristal. En effet, la présence d'une masselotte à l'extrémité de la languette va renforcer l'action du bras de levier constitué par ladite languette qui est destinée à pivoter puis à impacter le cristal au niveau de ladite masselotte.
Avantageusement, la languette est solidaire de l'organe de déclenchement, de sorte que son déplacement avant sa libération est dicté par le mouvement imprimé à l'organe de déclenchement.

Selon un mode de réalisation préférée de l'invention, l'organe de déclenchement est un bouton poussoir destiné à être actionné manuellement et pouvant coulisser le long d'un corps central allongé, par simple pression, pour provoquer un déplacement en translation de la languette.
De façon préférentielle, le bouton poussoir est placé à l'une des extrémités du corps central pour faciliter son actionnement et plus spécialement, à l'extrémité opposée à la buse d'injection.

Préférentiellement, le corps central est creux et présente sur sa paroi latérale interne une saillie faisant office de butée pour la languette.
Avantageusement, la surface de contact de la saillie contre laquelle vient s'appuyer la masselotte est plane.

De façon avantageuse, une zone de rugosité située entre le bouton poussoir et le corps central permet d'accroître les forces de frottement entre ces deux éléments, en cas de coulissement de l'un sur l'autre.
De façon préférentielle, la zone de rugosité est constituée par l'emboîtement mutuel d'aspérités annulaires de l'un des deux éléments dans des rainures de l'autre élément prévues pour les recevoir, de façon à induire un niveau de pression minimal pour commencer à enfoncer le bouton poussoir. Cela évite un déclenchement intempestif à partir d'une sollicitation anodine.

Préférentiellement, le bouton poussoir dispose d'une sûreté sous la forme d'une butée escamotable empêchant tout mouvement en translation dudit bouton. L'autre fonction de cette sûreté est de protéger l'extrémité de la seringue par laquelle doit s'éjecter le produit actif.
Avantageusement, la butée escamotable est constituée par un bouchon muni d'une collerette détachable, ledit bouchon assurant la protection de l'extrémité sensible de la seringue avant usage, et ladite collerette bloquant le bouton poussoir. Une ligne de faiblesse circulaire permet de dissocier la collerette du bouchon. La collerette est rigide et comporte une tirette pour la déchirer.

Selon un deuxième mode de réalisation préférée de l'invention, l'organe de déclenchement est un bouton destiné à être actionné manuellement par rotation autour d'un corps central, creux et allongé, de manière à provoquer la rotation de la languette, ledit corps présentant sur sa paroi latérale interne une saillie faisant office de butée pour ladite languette.
De façon avantageuse, la languette se termine par une masselotte.

De façon préférentielle, le bouton est placé à l'une des extrémités du corps central, pour faciliter le déclenchement manuel de la seringue.
Avantageusement, le bouton rotatif dispose d'une sûreté sous la forme d'une bague escamotable empêchant toute rotation dudit bouton.
Préférentiellement, la bague escamotable possède une tirette accessible manuellement pour déchirer puis enlever ladite bague.

Enfin, l'invention porte sur un dispositif de déclenchement d'une charge pyrotechnique comportant un organe de déclenchement relié à un moyen de percussion d'un cristal piézo-électrique, ledit moyen de percussion comprenant une languette maintenue sous contrainte élastique par blocage contre une butée et l'organe de déclenchement étant apte à déplacer ladite languette pour la libérer, de manière à venir impacter le cristal piézo-électrique. Avantageusement, la languette se termine par une masselotte pour accroître la force d'impact sur le cristal. Un tel dispositif de déclenchement peut, par exemple, être utilisé pour amorcer des grenades. En effet, l'utilisation d'un bouton pouvant être actionné par rotation ou par pression est adaptée à l'amorçage des grenades, dans la mesure où ce dispositif est moins contraignant à mettre en oeuvre que l'arrachement d'une goupille, surtout dans la situation particulière où la liberté de manoeuvre de l'utilisateur est limitée comme, par exemple, lorsqu'il porte des moufles. Ce dispositif offre en plus la possibilité d'un amorçage par percussion du bouton poussoir de la grenade sur une surface extérieure en dur. De façon générale, le dispositif de déclenchement selon l'invention, faisant intervenir une languette, est adapté à l'amorçage de tout type de charges pyrotechniques.

Les seringues sans aiguille selon l'invention présentent l'avantage de produire une réaction pyrotechnique moins violente que celle qui est perçue lors du fonctionnement d'une amorce à percussion. En effet, elles permettent une initiation en douceur de la composition génératrice de pression par l'intermédiaire d'un arc électrique ou d'une étincelle et non plus par l'intermédiaire d'une onde de choc.
De plus, lors de leur déclenchement et de leur fonctionnement elles engendrent un effet sonore extrêmement limité.
Enfin, elles présentent l'avantage d'être mises sur le marché civil sans subir les contraintes inhérentes aux dispositifs impliquant des explosifs ou des compositions détonantes.

On donne, ci-après, la description détaillée de trois modes de réalisation préférée de l'invention en se référant aux figures 1 à 6.
La figure 1 est une vue en coupe axiale longitudinale d'une seringue sans aiguille selon l'invention, faisant intervenir un bouton poussoir et une languette, et n'ayant pas encore fonctionné.
La figure 2 est une vue en coupe axiale longitudinale de la seringue de la figure 1, ayant fonctionné.
La figure 3 est une vue en coupe axiale longitudinale selon le plan III-III, d'une seringue selon l'invention, faisant intervenir un bouton rotatif et une languette et n'ayant pas encore fonctionné.
La figure 4 est une vue en coupe axiale transversale selon le plan IV-IV de la seringue de la figure 3.
La figure 5 est la même vue que celle de la figure 4 mais pour une seringue ayant fonctionné.
La figure 6 est une vue en coupe axiale longitudinale d'une seringue sans aiguille qui ne correspond pas à l'invention, faisant intervenir un bouton poussoir, un ressort et une masselotte, et n'ayant pas encore fonctionné.

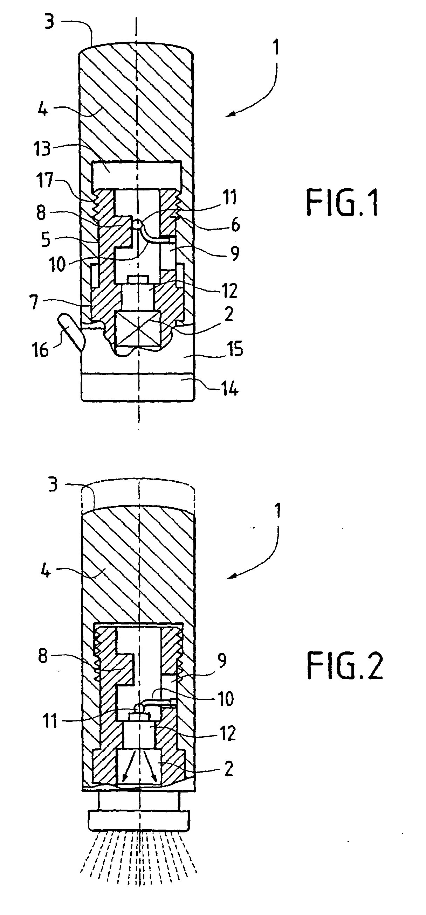

En se référant à la figure 1, selon le premier mode de réalisation préférée de l'invention, la seringue sans aiguille 1 présente une partie amont comprenant un dispositif de déclenchement et une partie aval comprenant une charge pyrotechnique 2, le principe actif sous forme solide ou liquide, une buse d'éjection et un guide d'application sur la peau. Le dispositif de déclenchement inclut un bouton poussoir 3, un moyen de percussion et un cristal piézo-électrique 12.

Le bouton poussoir 3 a une forme sensiblement cylindrique et est constitué par une partie pleine 4 prolongée par une partie cylindrique creuse 5 de diamètre extérieur identique. Cette partie cylindrique creuse 5 de diamètre extérieur constant comporte un épaulement interne permettant de distinguer un cylindre arrière creux de forte épaisseur en continuité avec un cylindre avant creux de plus faible épaisseur, le cylindre arrière étant compris entre la partie pleine 4 et le cylindre avant. Le cylindre arrière possède sur sa paroi latérale interne une zone filetée. La partie cylindrique creuse 5 enserre, sur une partie de sa longueur, un corps cylindrique creux 6 possédant sur sa paroi latérale externe une zone filetée, ledit corps 6 étant prolongé par une embase cylindrique élargie 7. La paroi latérale interne du corps cylindrique creux 6 comporte une saillie 8 dont la surface est plane, et une ouverture 9 est pratiquée dans la paroi dudit corps 6 dans une position diamétralement opposée à celle de la saillie plane 8. Ainsi, le canal interne du corps cylindrique creux 6 débouche, au niveau de cette ouverture 9, sur la paroi latérale interne de la partie cylindrique creuse 5 du bouton poussoir 3.

Solidarisée à la paroi latérale interne de la partie cylindrique creuse 5 du bouton poussoir 3, une languette 10 émerge de ladite ouverture 9, ladite languette 10 se terminant à son extrémité libre par une masselotte 11. En l'absence de toute contrainte, ladite languette 10 est implantée dans la paroi latérale interne de la partie cylindrique creuse 5, de façon à ce que sa position naturelle corresponde à une position pour laquelle la masselotte 11 se retrouve bien au dessous de son point d'implantation dans la paroi.

Lorsque le dispositif de déclenchement n'a pas encore fonctionné, toutes les pièces décrites ci avant sont agencées les unes par rapport aux autres de sorte que :
- le bouton poussoir 3 enserre le corps cylindrique creux 6 de façon à ce que leurs zones filetées s'emboîtent l'une dans l'autre et qu'un espace libre 13 subsiste entre la partie pleine 4 du bouton poussoir 3 et l'extrémité du corps cylindrique creux 6 lui faisant face. Il faut souligner que le terme «emboîter» est approprié à la situation puisqu'il ne s'agit pas d'un vissage en bonne et due forme du corps creux 6 dans la partie creuse 5 du bouton poussoir 3. Il s'agit plutôt de l'emboîtement d'une série de protubérances annulaires appartenant au corps creux 6 dans des rainures circulaires creusées dans la paroi latérale interne de la partie cylindrique creuse 5 du bouton poussoir 3, et réciproquement. Cet emboîtement a pour but de créer une zone de rugosité 17 constituée de points durs, de manière à accroître les forces de frottement en cas de coulissement du bouton poussoir 3 le long du corps cylindrique creux 6,
- la paroi latérale externe de l'embase élargie 7 du corps cylindrique creux 6 se retrouve au contact de la paroi latérale interne du cylindre avant de la partie cylindrique creuse 5 du bouton poussoir 3,
- l'extrémité du cylindre avant est repliée sur l'embase 7 de façon à éviter au bouton poussoir 3 d'être facilement retiré de la seringue 1,
- la languette 10 est déformée élastiquement par rotation autour d'un axe passant par le point de contact entre ladite languette 10 et la paroi latérale interne de la partie cylindrique 5 sur laquelle elle est implantée, cet axe étant à la fois perpendiculaire à l'axe de la languette 10 et à l'axe du corps cylindrique creux 6. Après avoir été déformée élastiquement, ladite languette 10 est bloquée contre la saillie plane 8, au niveau de la masselotte 11,
- la languette 10 sous contrainte émerge de l'ouverture 9 en étant au contact du bord de ladite ouverture 9 le plus proche de la partie pleine 4 du bouton poussoir 3.

Une charge pyrotechnique 2 cylindrique est fixée dans le corps cylindrique creux 6 de façon à ce que sa paroi latérale externe reste au contact de la paroi latérale interne dudit corps 6. Un cristal piézo-électrique 12 se retrouve placé entre la charge pyrotechnique 2 et la languette 10 sous contrainte, en étant au contact de l'une des deux faces circulaires de ladite charge 2. La seringue 1 dispose d'une sûreté sous la forme d'une butée escamotable constituée par un bouchon 14 muni d'une collerette rigide 15 détachable, ayant le même diamètre que le diamètre extérieur du bouton poussoir 3. Le bouchon 14 de forme cylindrique vient s'emboîter autour de l'extrémité sensible de la seringue 1 par laquelle sera expulsé le principe actif.

La collerette 15 qui est de forme cylindrique est solidaire du bouchon 14, et est bloquée entre ledit bouchon 14 et l'extrémité libre de la partie cylindrique creuse 5 du bouton poussoir 3.
Une zone de prédécoupage, sous la forme d'une rainure circulaire, est réalisée entre la collerette 15 et le bouchon 14 et une tirette 16 fixée à ladite collerette 15 peut être facilement saisie par l'utilisateur pour contribuer à détacher la collerette 15.

Le fonctionnement de cette variante préférée de seringue selon l'invention fait intervenir les étapes suivantes.
L'utilisateur se saisit de la tirette 16 et agit de façon à provoquer la séparation de la collerette 15 et du bouchon 14 suivant la ligne circulaire de prédécoupage. Une fois la collerette 15 enlevée, le bouchon 14 de protection est à son tour retiré et la seringue 1 se retrouve ainsi déverrouillée.

La partie aval de la seringue 1 est mise au contact de la peau du patient à traiter. L'utilisateur exerce alors une pression manuelle sur le bouton poussoir 3 au niveau de sa partie pleine 4, de façon à l'enfoncer. Pour ce faire, il doit fournir un effort pour vaincre les forces de frottement induites par la zone de rugosité 17. Lorsque le bouton poussoir 3 commence à coulisser le long du corps cylindrique creux 6, il entraîne un déplacement linéaire de la languette 10 sous contrainte qui lui est solidaire. L'extrémité de ladite languette 10 se terminant par la masselotte 11 glisse alors le long de la saillie 8 à la même vitesse que le déplacement du bouton poussoir 3. En se référant à la figure 2, en accentuant la pression sur le bouton poussoir 3, l'extrémité de la languette 10 arrive en bout de saillie 8 puis, lorsque le déplacement linéaire se poursuit, pivote brutalement pour retrouver sa position naturelle sans contrainte.

Ce pivotement brutal entraîne la mise en vitesse de la masselotte 11 qui vient percuter à grande vitesse le cristal piézo-électrique 12 placé au contact de la charge pyrotechnique 2. Le cristal 12 produit un arc électrique qui initie la charge pyrotechnique 2 dont la combustion va générer des gaz qui contribueront à éjecter le principe actif à travers la peau du patient. Le déplacement maximum du bouton poussoir 3 correspond à la venue en butée de l'épaulement interne de la partie cylindrique creuse 5 contre l'embase élargie 7 du corps cylindrique creux 6.

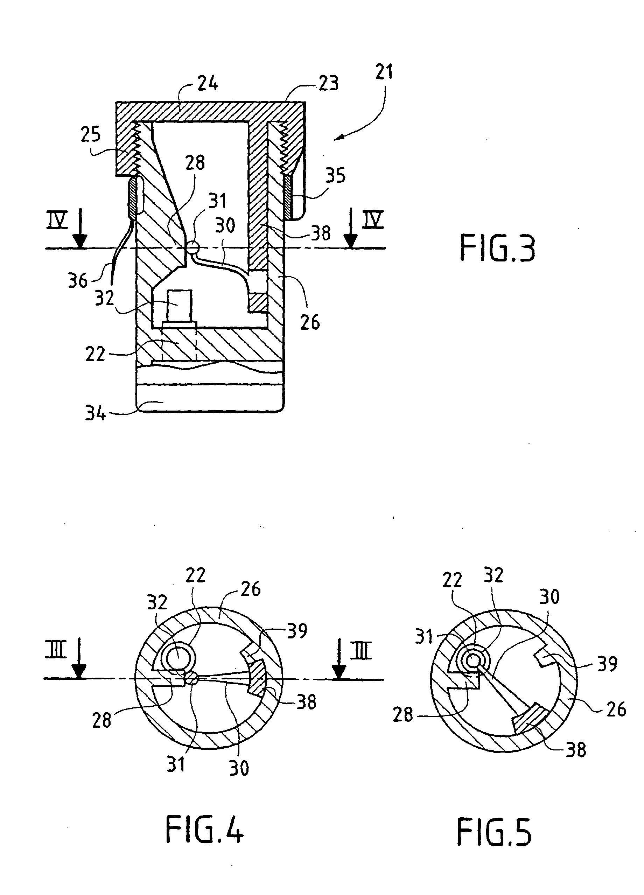

En se référant aux figures 3, 4 et 5, selon le deuxième mode de réalisation préféré de l'invention, la seringue 21 présente une partie amont comprenant un dispositif de déclenchement et une charge pyrotechnique 22, le principe actif sous forme solide ou liquide et une partie aval englobant une buse d'éjection et un guide d'application sur la peau. Le dispositif de déclenchement inclut un bouton 23 pouvant être actionné par rotation, un moyen de percussion et un cristal piézo-électrique 32. Le bouton 23 est constitué par une paroi latérale 25 cylindrique, filetée sur sa surface interne, et par une face circulaire plane 24 obturant l'une des deux extrémités de ladite paroi latérale 25. De plus le bouton 23 a la particularité de posséder une protubérance 38 ayant la forme d'une portion de cylindre définie par une longueur constante et qui résulterait de la découpe d'un cylindre complet suivant deux plans radiaux. Cette protubérance 38 dont la forme pourrait s'apparenter à celle d'un parallélépipède rectangle incurvé, est située sur la surface circulaire plane 24 du bouton 23 dans une position pour laquelle les génératrices de cette protubérance 38 sont parallèles à l'axe de rotation de ladite surface circulaire 24, et de sorte que ladite protubérance 38 soit disposée de façon concentrique par rapport à la paroi latérale 25 cylindrique dudit bouton 23. Ce bouton 23 est vissé autour d'un corps cylindrique creux 26 de diamètre extérieur constant et possédant, située à l'une de ses extrémités et sur sa surface externe, une zone filetée. La paroi interne du corps cylindrique creux 26 présentent deux saillies. L'une 39 de forme allongée, peu émergente et dont la longueur est voisine de celle de la protubérance 38, est disposée parallèlement à l'axe du corps cylindrique creux 26. L'autre 28 est plus marquée que la première 39, mais sa hauteur d'émergence dans le canal interne du corps cylindrique creux 26 reste inférieure au rayon dudit canal interne.
La protubérance 38 du bouton 23 porte, au voisinage de son extrémité libre, une languette 30 se terminant par une masselotte 31.
En l'absence de toute contrainte, ladite languette 30 est implantée dans la protubérance 38 de façon à ce que sa position naturelle corresponde à une position pour laquelle la masselotte 31 se retrouve bien au dessous de son point d'implantation dans la protubérance 38.

Lorsque le dispositif de déclenchement n'a pas encore fonctionné, toutes les pièces décrites ci avant sont agencées les unes par rapport aux autres de sorte que :
- le bouton 23 est vissé autour de l'extrémité filetée du corps cylindrique creux 26, et la protubérance 38 est placée à l'intérieur dudit corps cylindrique 26 et au contact de sa paroi latérale interne puisque la courbure de ladite protubérance 38 respecte celle de ladite paroi interne,
- la protubérance 38 solidaire du bouton 23 se retrouve en butée contre la saillie 39 la moins avancée mais sensiblement de même longueur, de manière à bloquer l'un des deux sens possibles de rotation dudit bouton 23,
- la languette 30 qui est faite en acier à ressort est déformée élastiquement par rotation autour d'un axe passant par le point de contact entre ladite languette 30 et la protubérance 38 qui la porte, cet axe étant à la fois perpendiculaire à l'axe de la languette 30 et à l'axe du corps cylindrique creux 26. Après avoir été déformée élastiquement, ladite languette est bloquée contre la saillie 28 du corps cylindrique creux 26 la plus avancée.

Une charge pyrotechnique 22 cylindrique est fixée dans le corps cylindrique creux 26 de façon à ce que sa paroi latérale externe reste au contact de la paroi latérale interne dudit corps 26.
Un cristal piézo-électrique 32 se retrouve placé entre la charge pyrotechnique 22 et la languette 30 sous contrainte, en étant au contact de l'une des deux faces circulaires de ladite charge 22.

La seringue 21 dispose d'une sûreté sous la forme d'une bague à languette de sécurité escamotable 35 enserrant le bouton 23 et empêchant toute rotation de celui-ci. Une tirette 36 fixée à ladite bague 35 peut être facilement saisie par l'utilisateur pour contribuer à déchirer la bague 35. Un bouchon 34 s'emboîte autour de l'extrémité sensible de la seringue 21 par laquelle sera expulsé le principe actif.

Le fonctionnement de cette variante préférée de seringue selon l'invention se décompose suivant les étapes suivantes.

L'utilisateur se saisit de la tirette 36 et agit de façon à provoquer la déchirure de la bague 35 dans le but de retirer celle-ci et ainsi déverrouiller la seringue 21. La partie aval de la seringue 21 est mise au contact de la peau du patient à traiter. L'utilisateur commence à tourner le bouton 23 dans le seul sens autorisé par la saillie 39 servant de butée à la protubérance 38. La rotation du bouton 23 entraîne la rotation de la protubérance 38 et donc celle de l'extrémité de la languette 30 se terminant par la masselotte 31 en appui contre la saillie 28.
En accentuant la rotation du bouton 23, l'extrémité de la languette 30 finit par se décaler de la saillie 28 contre laquelle elle était en appui. La languette 30 pivote alors brutalement pour retrouver sa position naturelle sans contrainte. Ce pivotement brutal entraîne la mise en vitesse de la masselotte 31 qui vient percuter à vitesse élevée le cristal piézo-électrique 32 placé au contact de la charge pyrotechnique 22. Le cristal 32 produit un arc électrique qui initie la charge pyrotechnique 22 dont la combustion va générer des gaz qui contribueront à éjecter le principe actif à travers la peau du patient.

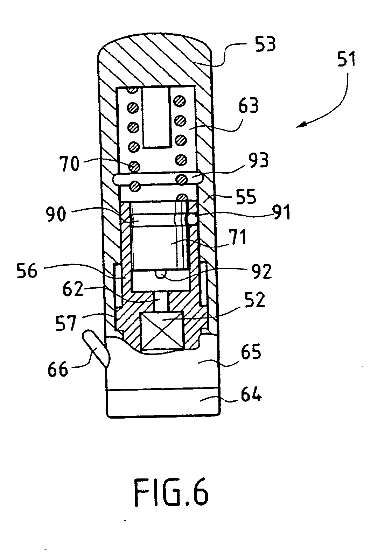

En se référant à la figure 6, la seringue 51 présente une partie amont comprenant un dispositif de déclenchement et une charge pyrotechnique 52, le principe actif sous forme solide ou liquide et une partie aval englobant une buse d'éjection et un guide d'application sur la peau. Le dispositif de déclenchement inclut un bouton poussoir 53, un moyen de percussion et un cristal piézo-électrique 62. Le bouton poussoir 53 est constitué par une paroi latérale 55 cylindrique de diamètre extérieur constant et présentant une extrémité fermée de forme arrondie. Ladite paroi 55 comporte un épaulement interne permettant de distinguer un cylindre arrière de forte épaisseur en continuité avec un cylindre avant de plus faible épaisseur, le cylindre arrière étant compris entre l'extrémité arrondie du bouton poussoir 53 et le cylindre avant. Cette paroi latérale 55 enserre sur une partie de sa longueur un corps cylindrique creux 56 de diamètre extérieur constant et possédant une embase cylindrique élargie 57. Ledit corps creux 57 présente un étranglement qui permet de diviser le canal interne dudit corps en trois zones, en continuité l'une de l'autre, chacune ayant un diamètre constant : une zone amont, la plus proche de l'extrémité arrondie du bouton poussoir 53, et dans laquelle est logée une masselotte 71 cylindrique présentant à sa périphérie une gorge circulaire 90, une zone intermédiaire de diamètre réduit constituant la zone d'étranglement dans laquelle est logé un cristal piézo-électrique 62, et une zone aval de diamètre agrandi par rapport à celui de la zone intermédiaire et dans laquelle est logée une charge pyrotechnique 52 de forme sensiblement cylindrique. Les parois latérales externes du cristal piézo-électrique 62 et de la charge pyrotechnique 52 sont au contact de la paroi interne du corps creux 56 qui les enserre, respectivement au niveau de la zone intermédiaire et de la zone aval. De plus, ledit cristal 62 et ladite charge 52 sont au contact l'un de l'autre.
De même, la paroi latérale externe de la masselotte 71 est au contact de la paroi latérale interne du corps creux 56 qui la loge au niveau de la zone amont, à l'exception toutefois de la partie de ladite masselotte 71 constituée par la gorge circulaire 90. La masselotte 71 présente deux faces circulaires planes dont l'une possède une aspérité centrale 92 qui lui est perpendiculaire. Le corps creux 56 comporte au niveau de la zone amont au moins un trou qui permet au canal interne dudit corps creux 56 de communiquer avec la paroi latérale interne 55 du bouton poussoir 53, ladite paroi étant au contact de la paroi latérale externe du corps creux 56. Chaque trou est occupé par une bille 91 dont le diamètre est supérieur à l'épaisseur de la paroi du corps creux 56 dans laquelle a été pratiqué le trou, ladite bille 91 étant coincée entre la paroi latérale interne 55 du bouton poussoir 53 et la gorge circulaire 90 de la masselotte 71. De façon avantageuse, le corps creux 56 possède une pluralité de trous alignés et régulièrement espacés à sa périphérie, chacun logeant une bille 91, de manière à mieux répartir les efforts s'exerçant sur la masselotte 71.

Cette bille 91 permet de bloquer la masselotte 71 dans le corps creux 56 de façon à ce que la face circulaire plane de ladite masselotte 71 ne possédant pas l'aspérité 92 affleure l'extrémité du corps cylindrique creux 56 dans lequel elle est logée. De cette manière, il subsiste un espace libre entre la face circulaire plane de la masselotte 71 portant l'aspérité 92 et le cristal piézo-électrique 62. Le bouton poussoir 53 enserre le corps cylindrique creux 56 de façon à ménager un espace libre 63 dans le cylindre arrière de la paroi latérale cylindrique 55 dudit bouton poussoir 53, ledit espace 63 étant délimité par la face interne de l'extrémité arrondie du bouton poussoir 53 et la surface circulaire plane comprenant la face circulaire plane de la masselotte 71 affleure ne possédant pas l'aspérité 92 et la bande circulaire du corps cylindrique creux 56 que la face de ladite masselotte 71 arase. Dans cet espace 63, la paroi latérale interne du bouton poussoir 53 est creusée par une gorge circulaire 93 lui permettant, à cet endroit, d'élargir son diamètre, et un ressort 70 vient en butée, d'une part, contre la face interne de l'extrémité arrondie du bouton poussoir 53 et, d'autre part, contre la face circulaire plane de la masselotte 71 opposée à celle possédant l'aspérité 92. La seringue 51 dispose d'une sûreté sous la forme d'une butée escamotable constituée par un bouchon 64 muni d'une collerette rigide 65 détachable, ayant le même diamètre que le diamètre extérieur du bouton poussoir 53. Le bouchon 64 de forme cylindrique vient s'emboîter autour de l'extrémité sensible de la seringue 51 par laquelle sera expulsé le principe actif. La collerette 65 qui est de forme cylindrique est solidaire du bouchon 64 et est bloquée entre ledit bouchon 64 et l'extrémité libre de la partie cylindrique creuse 55 du bouton poussoir 53.
Une zone de prédécoupage, sous la forme d'une rainure circulaire, est réalisée entre la collerette 65 et le bouchon 64 et une tirette 66 fixée à ladite collerette 65 peut être facilement saisie par l'utilisateur pour contribuer à détacher la collerette 65.

Le fonctionnement de cette variante de seringue fait intervenir les étapes suivantes.

L'utilisateur se saisit de la tirette 66 et agit de façon à provoquer la séparation de la collerette 65 et du bouchon 64 suivant la ligne circulaire de prédécoupage. Une fois la collerette 65 enlevée, le bouchon 64 de protection est à son tour retiré et la seringue 51 se retrouve ainsi déverrouillée.
La partie aval de la seringue 51 est mise au contact de la peau du patient à traiter. L'utilisateur exerce alors une pression manuelle sur le bouton poussoir 53 qui s'enfonce en coulissant autour du corps cylindrique creux 56. Le ressort 70 se comprime contre la masselotte 71 bloquée par les billes 91 tandis que la gorge circulaire 93 creusée dans la paroi latérale interne 55 du bouton poussoir 53 se rapproche de ladite bille 91. En accentuant la pression, ladite gorge 93 atteint le niveau de la bille 91 qui se dégage alors dans le nouvel espace offert par la gorge 93. La masselotte 71 qui n'est plus bloquée par les billes 91 mais qui est soumise à la pression du ressort 70 comprimé est violemment propulsée vers le cristal piézo-électrique 62. L'aspérité 92 de la masselotte 71 vient percuter ledit cristal 62 qui réagit en produisant un arc électrique.
La charge pyrotechnique 52 est alors initiée en combustion et les gaz produits vont contribuer à expulser le principe actif.

Le déplacement maximum du bouton poussoir 53 correspond à la venue en butée de l'épaulement interne de la paroi latérale cylindrique 55 contre l'embase élargie 57 du corps cylindrique creux 56.

## Revendications

1. Seringue sans aiguille munie d'un dispositif d'initiation comprenant un dispositif de déclenchement et une charge pyrotechnique (2, 22, 52), ledit dispositif de déclenchement incluant un cristal piézo-électrique (12, 32, 62) et un organe de déclenchement (3, 23, 53) relié à un moyen de percussion dudit cristal (12, 32, 62), **caractérisée en ce que** le moyen de percussion comprend une languette (10, 30) maintenue sous contrainte élastique par blocage contre une butée (8, 28) et l'organe de déclenchement (3, 23) est apte à déplacer ladite languette (10, 30) pour la libérer, de manière à venir impacter le cristal piézo-électrique (12, 32).

2. Seringue sans aiguille selon la revendication 1, **caractérisée en ce que** la languette (10, 30) se termine par une masselotte (11, 31) pour accroître la force d'impact sur le cristal (12, 32).

3. Seringue sans aiguille selon la revendication 1, **caractérisée en ce que** la languette (10, 30) est solidaire de l'organe de déclenchement (3, 23) de sorte que son déplacement avant sa libération est dicté par le mouvement imprimé à l'organe de déclenchement (3, 23).

4. Seringue sans aiguille selon la revendication 3, **caractérisée en ce que** l'organe de déclenchement (3) est un bouton poussoir destiné à être actionné manuellement et pouvant coulisser le long d'un corps central allongé (6), par simple pression, pour provoquer un déplacement en translation de la languette (10).

5. Seringue sans aiguille selon la revendication 4, **caractérisée en ce que** le corps central (6) est creux et présente sur sa paroi latérale interne une saillie (8) faisant office de butée pour la languette (10).

6. Seringue sans aiguille selon la revendication 4, **caractérisée en ce qu'**une zone de rugosité (17) située entre le bouton poussoir (3) et le corps central (6) permet d'accroître les forces de frottement entre ces deux éléments, en cas de coulissement de l'un sur l'autre.

7. Seringue sans aiguille selon la revendication 3, **caractérisée en ce que** l'organe de déclenchement (23) est un bouton destiné à être actionné manuellement par rotation autour d'un corps central (26) creux et allongé, de manière à provoquer la rotation de la languette (30), ledit corps (26) présentant sur sa paroi latérale interne une saillie (28) faisant office de butée pour ladite languette (30).

8. Seringue sans aiguille selon l'une quelconque des revendications 4 ou 7, **caractérisée en ce que** le bouton (3, 23) est placé à l'une des extrémités du corps central (6, 26).

9. Seringue sans aiguille selon la revendication 7, **caractérisée en ce que** le bouton (23) dispose d'une sûreté sous la forme d'une bague escamotable (35) empêchant toute rotation dudit bouton (23).

10. Seringue sans aiguille selon la revendication 4, **caractérisée en ce que** le bouton poussoir (3) dispose d'une sûreté sous la forme d'une butée escamotable empêchant tout mouvement en translation dudit bouton (3).

11. Seringue sans aiguille selon la revendication 10, **caractérisée en ce que** la butée escamotable est constituée par un bouchon (14) muni d'une collerette détachable (15).

12. Dispositif de déclenchement adapté à être utilisé avec la seringue définie à la revendication 1, **caractérisé en ce qu'**il comporte un organe de déclenchement (3, 23) relié à un moyen de percussion d'un cristal piézo-électrique (12, 32), ledit moyen de percussion comprenant une languette (10, 30) maintenue sous contrainte élastique par blocage contre une butée (8, 28) et l'organe de déclenchement (3, 23) étant apte à déplacer ladite languette (10, 30) pour la libérer, de manière à venir impacter le cristal piézo-électrique (12, 32).

13. Dispositif de déclenchement selon la revendication 12, **caractérisé en ce que** la languette (10, 30) se termine par une masselotte (11, 31) pour accroître la force d'impact sur le cristal (12, 32).

## Patentansprüche

1. Mit einer Zündvorrichtung versehene, nadellose Spritze, die eine Auslösevorrichtung und eine pyrotechnische Ladung (2, 22, 52) enthält, wobei die Auslösevorrichtung einen piezoelektrischen Kristall (12, 32, 62) und ein Auslöseorgan (3, 23, 53) enthält, das mit einem Schlagmittel für den Kristall (12, 32, 62) verbunden ist, **dadurch gekennzeichnet, dass** das Schlagmittel eine Zunge (10, 30) aufweist, die durch Blockieren gegen einen Anschlag (8, 28) unter elastischer Beanspruchung gehalten wird, und das Auslöseorgan (3, 23) in der Lage ist, die Zunge (10, 30) für ihre Freigabe zu verschieben, damit sie auf den piezoelektrischen Kristall (12, 32) aufprallt.

2. Nadellose Spritze nach Anspruch 1, **dadurch gekennzeichnet, dass** die Zunge (10, 30) in einem Schlagbolzen (11, 31) endet, um die Aufprallkraft auf den Kristall (12, 32) zu erhöhen.

3. Nadellose Spritze nach Anspruch 1, **dadurch gekennzeichnet, dass** die Zunge (10, 30) fest mit dem Auslöseorgan (3, 23) verbunden ist, so dass ihre Verschiebung vor ihrer Freigabe durch die dem Auslöseorgan (3, 23) verliehene Bewegung bedingt wird.

4. Nadellose Spritze nach Anspruch 3, **dadurch gekennzeichnet, dass** das Auslöseorgan (3) einen Druckknopf ist, der manuell betätigt wird und durch einfachen Druck entlang eines zentralen länglichen Körpers (6) gleiten kann, um eine Translationsverschiebung der Zunge (10) zu bewirken.

5. Nadellose Spritze nach Anspruch 4, **dadurch gekennzeichnet, dass** der zentrale Körper (6) hohl ist und auf seiner inneren Seitenwand einen Vorsprung (8) aufweist, der als Anschlag für die Zunge (10) dient.

6. Nadellose Spritze nach Anspruch 4, **dadurch gekennzeichnet, dass** eine Unebenheitszone (17), die sich zwischen dem Druckknopf (3) und dem zentralen Körper (6) befindet, es ermöglicht, die Reibungskräfte zwischen diesen beiden Elementen bei ihrem Gleiten aufeinander zu erhöhen.

7. Nadellose Spritze nach Anspruch 3, **dadurch gekennzeichnet, dass** das Auslöseorgan (23) ein Knopf ist, der manuell durch Drehung um einen hohlen und länglichen zentralen Körper (26) betätigbar ist, um die Drehung der Zunge (30) zu bewirken, wobei der Körper (26) auf seiner inneren Seitenwand einen Vorsprung (28) aufweist, der als Anschlag für die Zunge (30) dient.

8. Nadellose Spritze nach einem der Ansprüche 4 oder 7, **dadurch gekennzeichnet, dass** der Knopf (3, 23) an einem der Enden des zentralen Körpers (6, 26) angeordnet ist.

9. Nadellose Spritze nach Anspruch 7, **dadurch gekennzeichnet, dass** der Knopf (23) über eine Sicherheitseinrichtung in Form eines entfernbaren Rings (35) verfügt, der jede Drehung des Knopfes (23) verhindert.

10. Nadellose Spritze nach Anspruch 4, **dadurch gekennzeichnet, dass** der Druckknopf (3) über eine Sicherheitseinrichtung in Form eines entfernbaren Anschlags verfügt, der jede Translationsbewegung des Knopfs (3) verhindert.

11. Nadellose Spritze nach Anspruch 10, **dadurch gekennzeichnet, dass** der entfernbare Anschlag von einem Stopfen (14) gebildet wird, der mit einem lösbaren Kragen (15) versehen ist.

12. Auslösevorrichtung, die ausgelegt ist, um mit der in Anspruch 1 definierten Spritze zusammenzuwirken, **dadurch gekennzeichnet, dass** sie ein Auslöseorgan (3, 23) aufweist, das mit einem Schlagmittel für einen piezoelektrischen Kristall (12, 32) verbunden ist, wobei das Schlagmittel eine Zunge (10, 30) aufweist, die durch Blockieren gegen einen Anschlag (8, 28) unter elastischer Beanspruchung gehalten wird, und das Auslöseorgan (3, 23) in der Lage ist, die Zunge (10, 30) für ihre Freigabe zu verschieben, damit sie auf den piezoelektrischen Kristall (12, 32) aufprallt.

13. Auslösevorrichtung nach Anspruch 12, **dadurch gekennzeichnet, dass** die Zunge (10, 30) in einem Schlagbolzen (11, 31) endet, um die Aufprallkraft auf den Kristall (12, 32) zu erhöhen.

## Claims

1. Needleless syringe provided with an initiating device comprising a triggering device and a pyrotechnic charge (2, 22, 52), the said triggering device including a piezo-electric crystal (12, 32, 62) and a triggering component (3, 23, 53) connected to a means for striking the said crystal (12, 32, 62), **characterized in that** the striking means comprises a tongue (10, 30) held under elastic stress by being locked against the stop (8, 28) and the triggering component (3, 23) is capable of moving the said tongue (10, 30) in order to free it, so as to impact the piezo-electric crystal (12, 32).

2. Needleless syringe according to claim 1, **characterized in that** the tongue (10, 30) ends in a flyweight (11, 31) so as to increase the force of impact on the crystal (12, 32).

3. Needleless syringe according to claim 1, **characterized in that** the tongue (10, 30) is secured to the triggering component (3, 23) so that its movement before being released is dictated by the movement imprinted on the triggering component (3, 23)

4. Needleless syringe according to claim 3, **characterized in that** the triggering component (3) is a push button designed to be actuated manually and that can slide along a central elongated body (6), by simple pressure, in order to bring about a movement of the tongue in translation (10).

5. Needleless syringe according to claim 4, **characterized in that** the central body (6) is hollow and has, on its inner lateral wall, a projection (8) acting as a stop for the tongue (10).

6. Needleless syringe according to claim 4, **characterized in that** a roughened zone (17) situated between the push button (3) and the central body (6) enables the frictional forces between these two elements to be increased, in the case where they slide on each other.

7. Needleless syringe according to claim 3, **characterized in that** the triggering component (23) is a button designed to be actuated manually by rotation about a hollow elongated central body (26), so as to bring about rotation of the tongue (30), the said body (26) having, on its inner lateral wall, a projection (28) acting as a stop for the said tongue (30).

8. Needleless syringe according to either of claims 4 or 7, **characterized in that** the button (3, 23) is placed at one of the ends of the central body (6, 26).

9. Needleless syringe according to claim 7, **characterized in that** the button (23) possesses a safety device in the form of a retractable ring (35) preventing any rotation of the said button (3).

10. Needleless syringe according to claim 4, **characterized in that** the push button (3) possesses a safety device in the form of a retractable stop preventing any movement of the said button (3) in translation.

11. Needleless syringe according to claim 10, **characterized in that** the retractable stop consists of a stopper (14) provided with a detachable collar (15).

12. Triggering device adapted so as to be used with the syringe defined in claim 1, **characterized in that** it includes a triggering component (3, 23) connected to a means for striking a piezo-electric crystal (12, 32), the said striking means comprising a tongue (10,30) held under elastic stress by being locked against the stop (8, 28) and the triggering component (3, 23) being capable of moving the said tongue (10, 30) in order to free it, so as to impact the piezo-electric crystal (12, 32).

13. Triggering device according to claim 12, **characterized in that** the tongue (10, 30) ends in a bobweight (11, 31) in order to augment the force of impact on the crystal (12, 32).
